# FASCICULE DE BREVET EUROPEEN

(11) **EP 2 496 296 B1**
(45) Date de publication et mention de la délivrance du brevet: **23.07.2014**
(21) Numéro de dépôt: 10792955.6
(22) Date de dépôt: 02.11.2010
(51) Int. Cl.: A61M 15/00, A61M 15/08

(54) **DISPOSITIF DE DISTRIBUTION DE PRODUIT FLUIDE**
SPENDER FÜR FLÜSSIGMATERIAL
FLUID MATERIAL-DISPENSING DEVICE

(30) Priorité: 03.11.2009 FR 0957774
(43) Date de publication de la demande: 12.09.2012
(73) Titulaire: Aptar France SAS, 27110 Le Neubourg (FR)
(72) Inventeur: PARDONGE, Jean-Marc, F-76520 Les Authieux sur Port Saint Ouen (FR)
(74) Mandataire: CAPRI
(86) Numéro de dépôt international: PCT/FR2010/052342
(87) Numéro de publication internationale: WO 2011/055068

(56) Documents cités:
- EP-A1- 1 369 139
- WO-A1-00/51672
- WO-A1-2008/077623

## Description

La présente invention concerne un dispositif de distribution de produit fluide, et plus particulièrement un dispositif pourvu d'un indicateur visuel de prise de doses.

Dans les dispositifs de distribution de produit fluide, qui utilisent des organes de distribution tels que des pompes ou des valves pour réaliser la distribution, le produit fluide distribué est généralement finement pulvérisé sous forme d'un spray. Dans le cas des dispositifs médicaux du type distributeurs de médicaments par voie nasale, la dose de produit distribuée peut être faible et le produit peut être distribué sous la forme d'un spray particulièrement fin. Dans ce cas, l'utilisateur ne perçoit pas toujours la distribution effective du produit. Divers systèmes de comptage de doses existent pour renseigner l'utilisateur sur le nombre de doses distribué ou restant à distribuer à l'intérieur du dispositif de distribution. Ces compteurs ou indicateurs sont toutefois généralement actionnés lors de l'actionnement du dispositif, de manière mécanique, et témoignent donc de l'actionnement du dispositif mais pas de la distribution effective du produit fluide à travers l'orifice de distribution. En cas de dysfonctionnement, la dose peut être comptée sans avoir été effectivement distribuée à l'utilisateur. Les documents WO 2008/077623 et WO 00/51672 décrivent des dispositifs de l'état de la technique.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide qui indique à l'utilisateur la prise de doses effective, c'est-à-dire la distribution effective du produit fluide à travers l'orifice de pulvérisation.

La présente invention a également pour but de fournir un dispositif de distribution de produit fluide qui assure une information fiable de l'utilisateur quant à la distribution effective de la dose.

La présente invention a également pour but de fournir un dispositif de distribution de produit fluide qui soit simple et peu coûteux à fabriquer et à réaliser.

La présente invention a donc pour objet un dispositif de distribution de produit fluide, comportant un organe de distribution, tel qu'une pompe ou une valve, monté sur un réservoir de produit fluide et actionné par une tête de distribution comportant un orifice de pulvérisation, ledit dispositif comportant des moyens d'indication visuelle mobiles entre une position d'indication et une position de non-indication, lesdits moyens d'indication visuelle étant déplacés en position de non-indication au début de chaque actionnement dudit organe de distribution, et déplacés de ladite position de non-indication vers ladite position d'indication par ledit produit fluide lors de sa distribution, lesdits moyens d'indication visuelle comprenant un organe mobile sollicité par un ressort vers sa position de non-indication et sollicité par la pression du produit fluide en cours de distribution vers sa position d'indication.

Avantageusement, lesdits moyens d'indication visuelle sont disposés à l'intérieur de ladite tête de distribution, et sont au moins partiellement visibles de l'extérieur en position d'indication.

Avantageusement, ledit organe mobile est bloqué en position d'indication par un élément de blocage sollicité élastiquement vers sa position de blocage.

Avantageusement, ledit élément de blocage est libéré de sa position de blocage en début d'actionnement de l'organe de distribution, permettant ainsi un retour de l'organe mobile vers sa position de non-indication sous l'effet dudit ressort.

Avantageusement, ledit élément de blocage comporte au moins une patte flexible pourvue d'un profil interne coopérant avec une projection radiale dudit organe mobile pour le retenir en position d'indication.

Avantageusement, ladite au moins une patte flexible comporte une projection d'extrémité coopérant en début d'actionnement de l'organe de distribution avec une came adaptée à déformer ladite au moins une patte flexible radialement vers l'extérieur, supprimant ladite coopération du profil interne de la patte flexible avec la projection radiale de l'organe mobile.

Avantageusement, ladite projection d'extrémité comporte une surface d'extrémité inférieure inclinée, coopérant avec la came en début d'actionnement pour déformer chaque patte flexible radialement vers l'extérieur, et une surface supérieure inclinée, coopérant avec la came après l'actionnement, pour déformer chaque patte flexible radialement vers l'intérieur lors du retour de la tête de distribution vers sa position de repos.

Avantageusement, ladite came est formée par une projection arrondie solidaire du réservoir, notamment solidaire de la bague de fixation qui fixe l'organe de distribution sur ledit réservoir.

Avantageusement, ledit organe mobile est creux et définit ledit orifice de pulvérisation du dispositif.

Avantageusement, ledit organe mobile se projette axialement hors de ladite tête de distribution en position d'indication, ladite partie projetée hors de la tête formant une indication visuelle visible de l'extérieur.

Avantageusement, ledit organe mobile contient un insert fixe par rapport audit organe mobile.

Avantageusement, ladite tête de distribution est une tête nasale comportant une extension axiale destinée à pénétrer dans une narine d'un utilisateur.

Ces caractéristiques et avantages et d'autres de la présente invention apparaîtront plus clairement au cours de la description détaillée suivante d'un mode de réalisation particulier de celle-ci, faite en référence aux dessins joints, donnés à titre d'exemples non limitatifs, et sur lesquels
- la figure 1 est une vue schématique en section transversale d'un dispositif de distribution de produit fluide selon un mode de réalisation avantageux de la présente invention, en position de non-indication,
- la figure 2 est une vue schématique partielle en perspective d'une partie du dispositif de la figure 1,
- la figure 3 est une vue similaire à celle de la figure 1 en position d'indication, avant actionnement de l'organe de distribution,
- la figure 4 est une vue similaire à celle de la figure 3 en tout début d'actionnement de l'organe de distribution, et
- la figure 5 est une vue similaire aux figures 3 et 4 après distribution de produit fluide.

La présente invention concerne un témoin mécanique d'expulsion de doses. La présente invention s'applique plus particulièrement aux distributeurs de produit fluide dans le domaine de la pharmacie, et notamment les distributeurs de type nasal, mais bien entendu la présente invention pourrait aussi s'appliquer à d'autres domaines d'application.

Le principe de l'invention consiste à prévoir un élément mécanique mobile qui est déplacé d'une position de non-indication vers une position d'indication par la pression du produit fluide au moment de sa distribution. Par position d'indication, on entend une position qui indique qu'une dose de produit fluide a effectivement été distribuée à travers l'orifice de pulvérisation lors du précédent actionnement du dispositif. Par position de non-indication, on entend une position qui indique, au contraire, l'absence de distribution effective d'une dose de produit fluide. Ainsi, après utilisation du dispositif, l'utilisateur voit le témoin dans la position d'indication, ce qui lui garantit que la dose a été distribuée. Le témoin reste ensuite en position d'indication jusqu'au prochain actionnement du dispositif, bloqué dans cette position par un élément de blocage. Au moment du prochain actionnement, lorsque l'utilisateur prend le dispositif en main, il voit le témoin en position d'indication. En tout début de la course d'actionnement du dispositif, le témoin est ramené vers sa position de non-indication. Il est à noter que dans le cas d'un dispositif de distribution de type nasal, dans lequel l'utilisateur introduit une extension nasale de la tête de distribution dudit dispositif dans sa narine, l'utilisateur ne voit donc pas le témoin revenir à sa position de non-indication en tout début de course d'actionnement. Ensuite, lorsque le produit sera effectivement distribué, généralement vers la fin de la course d'actionnement du dispositif, la pression du produit fluide en cours de distribution ramènera automatiquement ledit témoin en position d'indication. Ce passage par la position de non-indication en tout début d'actionnement garantit à l'utilisateur que la position d'indication signifie une distribution effective de la dose de produit fluide. Dans l'hypothèse où la dose ne serait pas émise, le témoin serait en effet maintenu en position de non-indication puisque n'étant pas ramené vers sa position d'indication par la pression du produit fluide en cours de distribution.

Les figures représentent un mode de réalisation particulier de la présente invention. Il est à noter que ce mode de réalisation n'est qu'un exemple et que l'invention n'est pas limitée à ce mode de réalisation. En particulier, les moyens d'indication visuelle 50 pourraient être réalisés différemment.

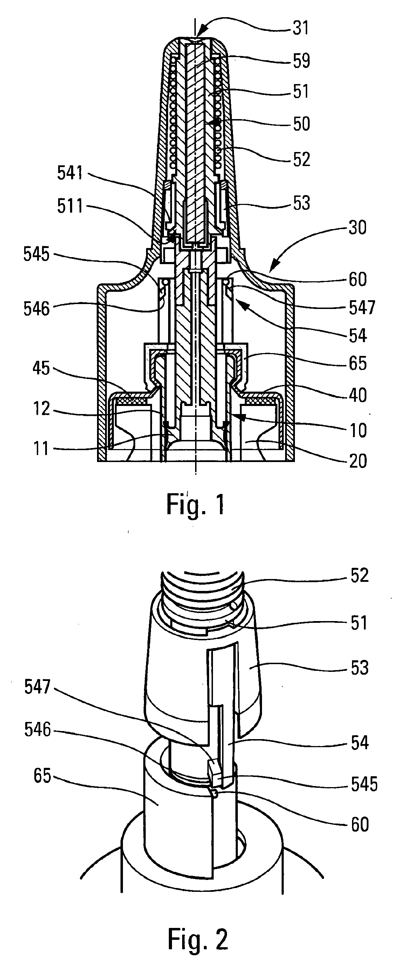

Dans le dispositif représenté sur la figure 1, un organe de distribution 10, qui en l'occurrence est une pompe, est monté sur un réservoir 20 (dont seulement le col est représenté) au moyen d'une bague de fixation 40, avec interposition d'un joint de col 45. Un piston 11 coulisse de manière étanche dans un corps de pompe 12, de manière classique. Bien entendu, la présente invention pourrait également s'appliquer à une pompe différente ou à une valve avec une soupape fonctionnant avec un gaz propulseur. L'organe de distribution, auquel on se réfèrera par la suite sous le terme de pompe 10, est actionné par une tête de distribution 30 qui est montée sur ladite pompe. Dans l'exemple représenté sur les figures, cette tête de distribution 30 est une tête de distribution nasale comportant une extension axiale, pourvue d'un orifice de pulvérisation 31, destinée à être insérée dans une narine.

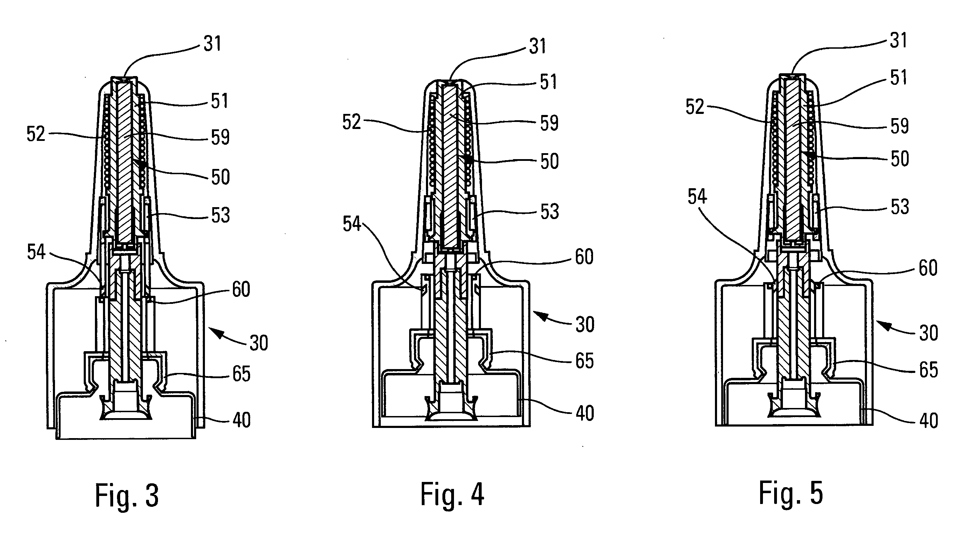

Selon l'invention, des moyens d'indication visuelle 50 sont prévus, de préférence disposés à l'intérieur de la tête de distribution 30. Dans le mode de réalisation représenté, ces moyens d'indication visuelle 50 comprennent un organe mobile 51 déplaçable entre une position d'indication et une position de non-indication. Dans la position d'indication, qui est représenté schématiquement sur les figures 3 et 5, ledit organe mobile 51 se projette axialement hors de la tête de distribution 30. Cette partie projetée hors de la tête forme alors des moyens d'indication visuelle visibles par l'utilisateur depuis l'extérieur. Par exemple, la surface latérale dudit organe mobile 51 pourrait être d'une couleur différente de la tête de sorte que l'utilisateur verrait très aisément si cet organe mobile se projette ou non hors de la tête. Bien entendu, on peut aussi imaginer une fenêtre de visualisation dans l'organe de distribution 30 et des repères visuels sur l'organe mobile, par exemple des parties de couleurs différentes, visibles à travers ladite fenêtre selon la position d'indication ou de non-indication dudit organe mobile 51. Cet organe mobile 51 est sollicité vers sa position de non-indication par un ressort 52. Un élément de blocage 53 est prévu pour maintenir l'organe mobile 51 en position d'indication malgré la force élastique exercée par le ressort 52. Cet élément de blocage 53 peut être formé par un manchon disposé radialement à l'extérieur dudit organe mobile 51 et qui est axialement fixe dans la tête de distribution 30. En d'autres termes, l'organe mobile 51 se déplace axialement à l'intérieur dudit élément de blocage 53. En position d'indication, ledit élément de blocage 53 coopère avec l'organe mobile 51 pour le retenir dans cette position. Avantageusement, l'élément de blocage 53 comporte au moins une patte flexible 54 pourvue d'un profil interne 541 adapté à coopérer avec une projection radiale externe 511 de l'organe mobile 51. Comme visible sur les figures 3 et 5, le profil interne 541 maintient l'organe mobile 51 en position d'indication. Par ailleurs, cette patte 54 est également adaptée à coopérer avec une came 60. Cette came 60 est adaptée à déformer la patte 54 radialement vers l'extérieur en tout début d'actionnement de la pompe 10. Plus précisément comme visible sur la figure 2, cette came 60 peut être formée par une projection, de préférence de forme arrondie, sur laquelle la patte 54 va s'appuyer via une projection d'extrémité 545, pourvue d'une surface d'extrémité inférieure inclinée 546, comme visible sur la figure 3. Dès le début de la course d'actionnement, la patte s'écartera donc radialement vers l'extérieur en glissant le long de la came 60. Cette déformation radiale désengagera la projection radiale 511 de l'organe mobile 51 dudit profil interne 541 de l'élément de blocage 53. Sous l'effet de la force du ressort 52, l'organe mobile 51 est alors ramené dans sa position de non-indication, représentée sur les figures 1 et 4, dans laquelle il n'est pas saillant hors de la tête de distribution. Lorsque l'organe mobile 51 est en position de non-indication, sa projection radiale 511 maintient la patte 54 de l'élément de blocage 53 radialement déformée vers l'extérieur. La course d'actionnement de la pompe se poursuit alors, et la pompe est actionnée pour distribuer une dose de produit fluide. Le produit fluide est alors expulsé sous pression à travers la tête de distribution et lorsque le produit fluide sous pression arrive au niveau de l'organe mobile 51, cette pression est suffisante pour ramener ledit organe mobile 51 en position d'indication, contre la force exercée par le ressort 52. Typiquement, les pressions de produit fluide à la sortie des pompes sont de l'ordre de 5 bars, voire plus. Lorsque l'organe mobile 51 est ramené en position d'indication, la patte 54 de l'élément de blocage 53 reprend élastiquement sa position de blocage. Dans la position actionnée de la tête de distribution, la projection d'extrémité 545 est disposée axialement de l'autre côté de la came 60. En fin d'actionnement, lorsque l'utilisateur relâche sa pression sur la tête de distribution, la tête de distribution remonte, notamment sous l'effet du ressort de rappel de la pompe. Lors de cette remontée, la patte flexible 54 coopère à nouveau avec la came 60 par une surface supérieure inclinée 547 de la projection d'extrémité 545, qui va déformer la patte flexible 54 radialement vers l'intérieur, pour maintenir le blocage de l'organe mobile 51 en position d'indication. L'organe mobile restera alors en position d'indication jusqu'au prochain actionnement. Avantageusement, l'élément de blocage 53 peut comporter deux pattes flexibles 54 diamétralement opposées.

Dans le mode de réalisation particulier représenté, l'organe mobile 51 est creux et définit le canal d'expulsion et l'orifice de pulvérisation 31 de la tête de distribution 30. Avantageusement, un insert 59 est disposé fixement à l'intérieur dudit organe mobile 51 et se déplace donc conjointement avec celui-ci entre les positions d'indication et de non-indication. De manière classique, cet insert 59 sert à définir un profil de pulvérisation (non représenté) au niveau de l'orifice de pulvérisation 31 pour rassurer une parfaite pulvérisation du produit fluide distribué.

Typiquement, avec une pompe de distribution nasale, le produit fluide est distribué après une course d'environ 3,5 mm à partir de la position de repos de la pompe. La présente invention procure le retour de l'organe mobile 51 vers la position de non-indication au tout début de la course d'actionnement, et avantageusement avant la fin du premier millimètre de course d'actionnement. Ainsi, il est garanti que l'organe mobile 51 est ramené en position de non-indication avant le début de la distribution de la dose de produit fluide. Ainsi, s'il y a un dysfonctionnement dans la pompe ou si l'actionnement n'est pas réalisé correctement, et que la dose n'est pas distribuée, l'organe mobile 51 restera en position de non-indication, pour informer l'utilisateur que la dose n'a pas été émise.

Bien que la présente invention a été décrite en référence à un mode de réalisation particulier de celle-ci, il est entendu que diverses modifications peuvent y être apportées sans sortir du cadre de la présente invention tel que défini par les revendications annexées.

## Revendications

1. Dispositif de distribution de produit fluide, comportant un organe de distribution (10), tel qu'une pompe ou une valve, monté sur un réservoir de produit fluide (20) et actionné par une tête de distribution (30) comportant un orifice de pulvérisation (31), **caractérisé en ce que** ledit dispositif comporte des moyens d'indication visuelle (50) mobiles entre une position d'indication et une position de non-indication, lesdits moyens d'indication visuelle (50) étant déplacés en position de non-indication au début de chaque actionnement dudit organe de distribution (10), et déplacés de ladite position de non-indication vers ladite position d'indication par ledit produit fluide lors de sa distribution, lesdits moyens d'indication visuelle (50) comprenant un organe mobile (51) sollicité par un ressort (52) vers sa position de non-indication et sollicité par la pression du produit fluide en cours de distribution vers sa position d'indication.

2. Dispositif selon la revendication 1, dans lequel lesdits moyens d'indication visuelle (50) sont disposés à l'intérieur de ladite tête de distribution (30), et sont au moins partiellement visibles de l'extérieur en position d'indication.

3. Dispositif selon la revendication 1 ou 2, dans lequel ledit organe mobile (51) est bloqué en position d'indication par un élément de blocage (53) sollicité élastiquement vers sa position de blocage.

4. Dispositif selon la revendication 3, dans lequel ledit élément de blocage (53) est libéré de sa position de blocage en début d'actionnement de l'organe de distribution (10), permettant ainsi un retour de l'organe mobile (51) vers sa position de non-indication sous l'effet dudit ressort (52).

5. Dispositif selon la revendication 4, dans lequel ledit élément de blocage (53) comporte au moins une patte flexible (54) pourvue d'un profil interne (541) coopérant avec une projection radiale (511) dudit organe mobile (51) pour le retenir en position d'indication.

6. Dispositif selon la revendication 5, dans lequel ladite au moins une patte flexible (54) comporte une projection d'extrémité (545) coopérant en début d'actionnement de l'organe de distribution (10) avec une came (60) adaptée à déformer ladite au moins une patte flexible (54) radialement vers l'extérieur, supprimant ladite coopération du profil interne (541) de la patte flexible (54) avec la projection radiale (511) de l'organe mobile (51).

7. Dispositif selon la revendication 6, dans lequel ladite projection d'extrémité (545) comporte une surface d'extrémité inférieure inclinée (546), coopérant avec la came (60) en début d'actionnement pour déformer chaque patte flexible (54) radialement vers l'extérieur, et une surface supérieure inclinée (547), coopérant avec la came (60) après l'actionnement, pour déformer chaque patte flexible radialement vers l'intérieur lors du retour de la tête de distribution (30) vers sa position de repos.

8. Dispositif selon la revendication 6 ou 7, dans lequel ladite came (60) est formée par une projection arrondie (65) solidaire du réservoir (20), notamment solidaire de la bague de fixation (40) qui fixe l'organe de distribution (10) sur ledit réservoir (20).

9. Dispositif selon l'une quelconque des revendications précédentes, dans lequel ledit organe mobile (51) est creux et définit ledit orifice de pulvérisation (31) du dispositif.

10. Dispositif selon la revendication 9, dans lequel ledit organe mobile (51) se projette axialement hors de ladite tête de distribution (30) en position d'indication, ladite partie projetée hors de la tête formant une indication visuelle visible de l'extérieur.

11. Dispositif selon la revendication 9 ou 10, dans lequel ledit organe mobile (51) contient un insert (59) fixe par rapport audit organe mobile (51).

12. Dispositif selon l'une quelconque des revendications précédentes, dans lequel ladite tête de distribution est une tête nasale comportant une extension axiale destinée à pénétrer dans une narine d'un utilisateur.

## Patentansprüche

1. Ausgabevorrichtung für ein fluides Produkt, aufweisend eine Ausgabeeinrichtung (10), wie eine Pumpe oder ein Ventil, die auf einem Behälter für ein fluides Produkt (20) angebracht ist und durch einen Ausgabekopf (30) betätigt wird, der eine Zerstäubungsöffnung (31) aufweist, **dadurch gekennzeichnet, dass** die Vorrichtung Sichtanzeigemittel (50) aufweist, die zwischen einer Anzeigeposition und einer Position, in der keine Anzeige erfolgt, beweglich sind, wobei die Sichtanzeigemittel (50) zu Beginn jeder Betätigung der Ausgabeeinrichtung (10) in die Position, in der keine Anzeige erfolgt, verschoben werden und durch das fluide Produkt bei dessen Ausgabe von der Position, in der keine Anzeige erfolgt, in die Anzeigeposition verschoben werden, wobei die Sichtanzeigemittel (50) eine mobile Einrichtung (51) aufweisen, die durch eine Feder (52) gegen ihre Position, in der keine Anzeige erfolgt, belastet wird und durch den Druck des fluiden Produktes während der Ausgabe gegen ihre Anzeigeposition belastet wird.

2. Vorrichtung nach Anspruch 1, wobei die Sichtanzeigemittel (50) im Inneren des Ausgabekopfes (30) angeordnet sind und in der Anzeigeposition zumindest teilweise von außen sichtbar sind.

3. Vorrichtung nach Anspruch 1 oder 2, wobei die mobile Einrichtung (51) in der Anzeigeposition von einem Sperrelement (53) gesperrt ist, das elastisch gegen seine Sperrposition belastet ist.

4. Vorrichtung nach Anspruch 3, wobei das Sperrelement (53) zu Beginn der Betätigung der Ausgabeeinrichtung (10) aus seiner Sperrposition gelöst wird, wodurch unter Einwirkung der Feder (52) eine Rückkehr der mobilen Einrichtung (51) in ihre Position, in der keine Anzeige erfolgt, ermöglicht wird.

5. Vorrichtung nach Anspruch 4, wobei das Sperrelement (53) mindestens eine flexible Lasche (54) aufweist, die mit einem Innenprofil (541) versehen ist, das mit einem radialen Vorsprung (511) der mobilen Einrichtung (51) zusammenwirkt, um diese in der Anzeigeposition zu halten.

6. Vorrichtung nach Anspruch 5, wobei die mindestens eine flexible Lasche (54) einen Endvorsprung (545) aufweist, der zu Beginn der Betätigung der Ausgabeeinrichtung (10) mit einem Nocken (60) zusammenwirkt, der dazu geeignet ist, die mindestens eine flexible Lasche (54) radial nach außen zu verformen, wobei die Zusammenwirkung des Innenprofils (541) der flexiblen Lasche (54) mit dem radialen Vorsprung (511) der mobilen Einrichtung (51) unterdrückt wird.

7. Vorrichtung nach Anspruch 6, wobei der Endvorsprung (545) eine untere geneigte Endfläche (546), die zu Beginn der Betätigung mit dem Nocken (60) zusammenwirkt, um jede flexible Lasche (54) radial nach außen zu verformen, und eine obere geneigte Oberfläche (547) aufweist, die nach der Betätigung mit dem Nocken (60) zusammenwirkt, um bei der Rückkehr des Ausgabekopfes (30) in seine Ruheposition jede flexible Lasche radial nach innen zu verformen.

8. Vorrichtung nach Anspruch 6 oder 7, wobei der Nocken (60) durch einen abgerundeten Vorsprung (65) einstückig mit dem Behälter (20) gebildet ist, insbesondere mit dem Befestigungsring (40) einstückig gebildet ist, der die Ausgabeeinrichtung (10) auf dem Behälter (20) hält.

9. Vorrichtung nach einem der vorhergehenden Ansprüche, wobei die mobile Einrichtung (51) hohl ist und die Zerstäubungsöffnung (31) der Vorrichtung begrenzt.

10. Vorrichtung nach Anspruch 9, wobei die mobile Einrichtung (51) in der Anzeigeposition axial aus dem Ausgabekopf (30) herauskragt, wobei der aus dem Kopf herauskragende Teil eine Sichtanzeige bildet, die von außen sichtbar ist.

11. Vorrichtung nach Anspruch 9 oder 10, wobei die mobile Einrichtung (51) einen Einsatz (59) enthält, der in Bezug auf die mobile Einrichtung (51) fixiert ist.

12. Vorrichtung nach einem der vorhergehenden Ansprüche, wobei der Ausgabekopf ein Nasenkopf ist, der eine axiale Erstreckung aufweist, die dafür gestaltet ist, in das Nasenloch eines Benutzers eingeführt zu werden.

## Claims

1. A fluid dispenser device comprising a dispenser member (10), such as a pump or a valve, that is mounted on a fluid reservoir (20) and that is actuated by a dispenser head (30) that includes a spray orifice (31), said device being **characterized in that** it further comprises visual indicator means (50) that are movable between an indicating position and a non-indicating position, said visual indicator means (50) being moved into their non-indicating position at the start of each actuation of said dispenser member (10), and being moved from said non-indicating position towards said indicating position by said fluid, while it is being dispensed, said visual indicator means (50) including a movable member (51) that is urged by a spring (52) towards its non-indicating position, and that is urged by the pressure of the fluid during dispensing towards its indicating position.

2. A device according to claim 1, wherein said visual indicator means (50) are arranged inside said dispenser head (30), and can be seen, at least in part, from the outside, in their indicating position.

3. A device according to claim 1 or claim 2, wherein said movable member (51) is blocked in the indicating position by a blocking element (53) that is urged resiliently towards its blocking position.

4. A device according to claim 3, wherein said blocking element (53) is released from its blocking position at the start of actuation of the dispenser member (10), thereby enabling the movable member (51) to return to its non-indicating position, under the effect of said spring (52).

5. A device according to claim 4, wherein said blocking element (53) includes at least one flexible tab (54) that is provided with an inner profile (541) that co-operates with a radial projection (511) of said movable member (51) so as to hold said movable member in its indicating position.

6. A device according to claim 5, wherein said at least one flexible tab (54) includes an end projection (545) that, at the start of actuation of the dispenser member (10), co-operates with a cam (60) that is adapted to deform said at least one flexible tab (54) radially outwards, eliminating said co-operation between the inner profile (541) of the flexible tab (54) and the radial projection (511) of the movable member (51).

7. A device according to claim 6, wherein said end projection (545) includes a sloping bottom end surface (546) that co-operates with the cam (60) at the start of actuation so as to deform each flexible tab (54) radially outwards, and a sloping top surface (547) that co-operates with the cam (60) after actuation so as to deform each flexible tab radially inwards while the dispenser head (30) is returning to its rest position.

8. A device according to claim 6 or claim 7, wherein said cam (60) is formed by a rounded projection (65) that is secured to the reservoir (20), in particular that is secured to the fastener ring (40) that fastens the dispenser member (10) on said reservoir (20).

9. A device according to any preceding claim, wherein said movable member (51) is hollow and defines said spray orifice (31) of the device.

10. A device according to claim 9, wherein said movable member (51) projects axially out from said dispenser head (30) in the indicating position, said portion projecting out from the head forming a visual indicator that can be seen from the outside.

11. A device according to claim 9 or claim 10, wherein said movable member (51) contains an insert (59) that cannot move relative to said movable member (51).

12. A device according to any preceding claim, wherein said dispenser head is a nasal head including an axial extension for penetrating into a user's nostril.
